# EUROPEAN PATENT SPECIFICATION

(11) **EP 3 588 052 B1**
(45) Date of publication and mention of the grant of the patent: **31.03.2021**
(21) Application number: 18180665.4
(22) Date of filing: 29.06.2018
(51) Int. Cl.: G01N 1/12, C21C 5/46, G01N 33/205, F27D 19/00, F27D 3/16

(54) **IMMERSION PROBE AND MEASURING SYSTEM FOR USE IN A CONVERTER STEELMAKING PROCESS**
TAUCHFÜHLER UND MESSSYSTEM ZUR VERWENDUNG IN EINEM KONVERTER STAHLVERFAHREN
SONDE A IMMERSION ET SYSTEME DE MESURE POUR UTILISATION DANS UN CONVERTISSEUR

(43) Date of publication of application: 01.01.2020
(73) Proprietor: Heraeus Electro-Nite International N.V., 3530 Houthalen (BE)
(72) Inventor: Beyens, Dries, 3530 Houthalen (BE); Neyens, Guido Jacobus, 3530 Houthalen (BE)
(74) Representative: Heraeus IP

(56) References cited:
- CN-U- 205 603 624
- US-A- 4 566 343
- US-A- 5 241 262
- US-A1- 2011 219 889

## Description

### FIELD OF THE INVENTION

The invention relates to an immersion probe for use in a converter steelmaking process. The invention also relates to a measuring system for use in a converter steelmaking process comprising a lance and an immersion probe.

### BACKGROUND OF THE INVENTION

In the steelmaking process, several process parameters of the molten metal are measured by means of a lance. In a typical converter process, at least two measurement are conducted. The first measurement is typically done at the moment the process model expects a carbon content of approximately 0.35%. A first immersion probe used for the first measurement typically comprises a combined sensor that measures the temperature of the molten metal, offers a liquidus measurement of the molten metal and retrieves a solid sample. The first immersion probe is sometimes called an inblow immersion probe, because the immersion is done during the blowing process of oxygen into the liquid steel for lowering the carbon content of the liquid steel. Based on the measurement results of the first immersion probe, the remaining blowing time is calculated. After the end of the blowing process a second immersion probe is typically immersed into the bath, offering the temperature, oxygen content and a solid sample. This second immersion probe is called an endblow immersion probe. The typical time between inblow time and the endblow time is in the range of 2 to 3 minutes.

A typical lance that is used in the steelmaking process is made from a metal material, whereas a typical immersion probe comprises a tube of paperboard which is telescoped over an immersion end portion of the lance with a force fit until the end of the tube engages a shoulder and/or coupling tap on the lance. The end of the tube that is directed towards the molten metal carries the actual sensing elements, such as thermocouples, and/or samplers comprising a sample chamber for taking a sample of the molten metal.

For taking a measurement and/or a sample of the molten metal the immersion probe is attached to the lance and vertically immersed into the converter adjacent to an oxygen supply lance. Immersing the lance with an attached immersion probe into the molten metal typical causes a lot of vibration to the lance and immersion probe, resulting in a small gap between the end of the tube and the immersion end portion of the lance. This gap is prone to fill with splashes of slag and steel during immersion and to slowly build up skull. Over time, skull will continue to grow until an immersion probe can't be reliably connected to the lance anymore. As a result thereof, improper reading will be obtained. In the worst case, the lance must be replaced with a new lance.

The problem of skull formation on the lance is known in the prior art. For example, the use of an elastic ring seal between the immersion end of the lance and the tube to reduce the deposition of frozen metal at the joint between lance and tube is described in US4566343A, CN205603642U, US5241262A and US2011/219889.

Even though the deposit of frozen metal can be reduced by means of an elastic ring seal, the elastic ring seal wears out quickly, and hence the residence time in the converter is shortened. To overcome this issue the elastic ring seal is often over-dimensioned in terms of increased wall-thickness, which results in low deformation of the elastic ring seal and allows high residence times in the converter. Unfortunately, using such an over-dimensioned elastic ring seal will cause small gaps between the immersion end of the lance and the tube. These small gaps are then again subject to penetration with slag and steel.

Therefore, the present invention is directed to an improved solution of the problem of how to prevent the deposition of frozen metal at the joint between the probe and the lance.

### SUMMARY OF THE INVENTION

The invention provides an immersion probe for use in a converter steelmaking process, comprising:
a tube having a mounting portion for mounting the tube to a lance,
wherein the mounting portion is adapted to couple to a coupling tap on an immersion end of the lance; and two elastic ring seals,
wherein the two elastic ring seals are arranged around the mounting portion of the tube,
wherein the two elastic ring seals are arranged circumferentially on top of each other for sealing a space between the coupling tap and the end portion of the immersion probe, and
wherein the outer circumference of an inner elastic ring seal which is in direct contact with the tube is at least partly in contact with the inner circumference of an outer elastic ring seal so that the outer elastic ring is configured to at least partly cover the inner elastic ring.

The tube can be made of paperboard similar to the tubes that are already known in the prior art. The mounting portion of the tube can be arranged on a first end of the tube and a sensing element and/or sampler can be arranged on a second end of the tube which is opposite the first end. According to the invention, the mounting portion is adapted to couple to a coupling tap on an immersion end of the lance. Therefore, the mounting portion can comprise coupling means, for example the coupling means that are shown in US 4,566,343, where both portions are coupled together via a force fit or push fit coupling. In one example, the mounting portion is simply an end portion of the tube.

According to the invention, the immersion probe comprises two elastic ring seals, wherein the two elastic ring seals are arranged around the mounting portion of the tube, and wherein the two elastic ring seals are arranged circumferentially on top of each other for sealing a space between the coupling tap and the end portion of the immersion probe.

Here, the term 'on top of each other' is used to refer to an arrangement of two elastic ring seals where the outer circumference of an inner elastic ring seal which is in direct contact with the tube is at least partly in contact with the inner circumference of an outer elastic ring seal so that the outer elastic ring at least partly covers the inner elastic ring.

The two elastic ring seals can have the same length and thickness, or can have different lengths and thicknesses. For example, the outer elastic ring seal can have a slightly larger diameter than the inner elastic ring seal to provide a proper fit. The two elastic ring seals can be in the shape of a closed ring but can also have free ends which overlap. The two elastic ring seals can attach to the tube in any convenient manner such as by glue or staples which do not interfere with the use of the elastic ring seals.

The space between the coupling tap and the end portion of the immersion probe can be a shoulder which can comprise an essentially planar surface on the lance which surrounds the coupling tap. When the tube is mounted onto the lance the two elastic ring seals can rest against the shoulder and seal the space between the coupling tap and the end portion of the immersion probe.

It has been advantageously found that by employing two elastic ring seals that are arranged on top of each other burning/destruction of the elastic ring seals takes place in phases. The inner ring seal will start to deteriorate after the outer ring seal is almost completely destroyed. Advantageously, this arrangement provides improved skull prevention and an improved residence time during the immersion in molten metal.

In one example of the invention, the mounting portion comprises a cylindrical opening for accommodating the coupling tap.

For example, the cylindrical opening can be an opening at the end of a rolled tube made from paperboard. An essentially cylindrical coupling tap which fits the geometry of the cylindrical opening can then be pushed into the cylindrical opening. The resulting force fit coupling holds the rolled tube on the lance. However, in examples of the invention an alternative coupling mechanism could be also used such as a bayonet mount.

In a further example of the invention, the two elastic ring seals are arranged at least partly in a recess around the mounting portion of the tube.

The recess, can be a groove located at the end portion of the tube that extends partly or fully around the circumference of the tube. In another example, the end portion of the tube can be tapered towards the end of the tube to allow installing the two elastic ring seals. Advantageously, by installing the two elastic ring seals in the recess, the elastic ring seals can be firmly held in place.

In an example, the tube can comprise at least one ventilation opening extending through the material of the tube and arranged in the recess and at least partly covered by at least one of the two elastic ring seals.

The ventilation openings allow gases and vapors from the molten metal which rise up in the tube to escape. Also, due to the ventilation openings the tube can be more easily fitted onto the lance, since pressure that might build up during the installation of the tube onto the lance can be ventilated. Advantageously, the two elastic ring seals protect the ventilation openings from clogging due to impurities such as splashes from steel and slag during the oxygen blowing process. Devices known from the prior art apply ventilation openings located below the shoulder of the lance in the immersion direction, i.e. seen when mounted onto the lance. To prevent steel and slag ingress, these ventilation openings are foreseen with metal springs that freeze before the liquid material can fill the gap between the immersion probe and the lance.

In an example, the tube comprises two to six ventilation openings, preferably the ventilation openings are having a diameter of 5 to 8 mm and are arranged in the recess.

By moving the ventilation openings away from the immersion end, the risk of steel and slag penetration will be reduced and the ventilation openings will be protected from splashes by means of the elastic ring seals. The dual layer formed by the elastic ring seals will increase the quality of the resulting covering means during the immersion, while the inner elastic ring seal can be considered intact as long as the outer elastic ring seal is present. The needed ventilation area is still available.

In another example, the two elastic ring seals are adapted to radially flush with an outer surface of the tube. For example, the thickness of the elastic ring seals can be chosen so that the two ring seals, when mounted according to the invention, flush with the outer surface of the tube. Here, the recess can be a countersunk hole that accommodates the two elastic ring seals. This assembly generates the benefit of easy packaging of the immersion probe for transportation on one hand and automatic manipulators often have a maximum outer diameter for immersion probes used therewith.

In an example, at least one of the elastic ring seals is adapted to extend beyond an end of the tube, preferably the two elastic ring seals are adapted to extend beyond the end of the tube.

The height of the elastic ring seals can be chosen so that at least one of the elastic ring seals overlaps the end of the tube to provide an improved sealing effect between tube and lance. In an example thereof, at least one elastic ring seal is adapted, preferably the two elastic ring seals are adapted, to extend beyond an end of the tube by 2 to 7 mm, preferably by 3 to 6 mm, more preferably by 4 to 5 mm.

In another example, the tube comprises at least a cardboard material.

For example, the tube can be made from a sheet of cardboard or paperboard that is wound in multiple layers to achieve a rigid tube. In alternative examples of the invention, the tube can comprise cellular plastic material or metal foils, often used for the purpose of overcoming humidity problems.

In one example, the tube having an outer diameter of approximately 80 mm.

A diameter of approximately 80 mm is required to connect the tube to most lances that are used in steel plants. However, in alternative examples, a wider or smaller diameter can be used depending on the diameter of the lance to which the tube shall be connected to.

In a further example, the two elastic ring seals comprise a rubber material.

Rubber material, such as natural rubber, synthetic rubber, neoprene rubber, nitrile rubber, cellular rubber, PVC, or silicone rubber, has good sealing properties, can withstand high temperatures, and is sufficiently inexpensive.

In an example, the two elastic ring seals comprise a material comprising ethylene propylene diene monomer, EPDM, rubber.

In another example, the two elastic ring seals have a length of 25 mm, and/or a thickness of 2.5 mm.

In yet another example, at least one of the elastic ring seals, preferably the two elastic ring seals, has a hardness of less than 40 shore.

Advantageously, a hardness of less than 40 shore allows a deformation of the two elastic ring seals by at least 50% when the tube is telescoped onto the coupling tap of the lance. It has been found that harder materials don't sufficiently compensate local deformations which are often found on coupling taps of lances. Advantageously, by employing elastic ring seals having a hardness of less than 40 shore allows to reliably cover/fill all gaps between the coupling tap of the lance and the mounting portion of the immersion probe.

The invention also relates to a measuring system for use in a converter steelmaking process comprising:
a tube having a mounting portion for mounting the tube to a lance,
wherein the mounting portion is adapted to couple to a coupling tap on an immersion end of the lance; and two elastic ring seals,
wherein the two elastic ring seals are arranged around the mounting portion of the tube,
wherein the two elastic ring seals are arranged circumferentially on top of each other for sealing a space between the coupling tap and the end portion of the immersion probe, and
wherein the outer circumference of an inner elastic ring seal which is in direct contact with the tube is at least partly in contact with the inner circumference of an outer elastic ring seal so that the outer elastic ring is configured to at least partly cover the inner elastic ring.

The immersion probe can be the immersion probe which is defined in the various examples above.

### BRIEF DESCRIPTION OF THE DRAWINGS

The following schematic drawings show aspects of the invention for improving the understanding of the invention in connection with some exemplary illustrations, wherein
- Figure 1a: shows a sectional view of an immersion probe for use in a converter steelmaking process according to a first embodiment of the invention;
- Figure 1b: shows a sectional view of an immersion probe for use in a converter steelmaking process according to a second embodiment of the invention;
- Figure 2a: shows a sectional view of a measurement system for use in a converter steelmaking process comprising a lance and an immersion probe according to the second embodiment of the invention mounted onto the lance;
- Figure 2b: shows a sectional view of a measurement system for use in a converter steelmaking process comprising a lance and an immersion probe according to a third embodiment of the invention mounted onto the lance; and
- Figures 3a, 3b: show cross-sectional views taken along section lines 'A' which are shown in Figures 2a and 2b.

### DETAILED DESCRIPTION

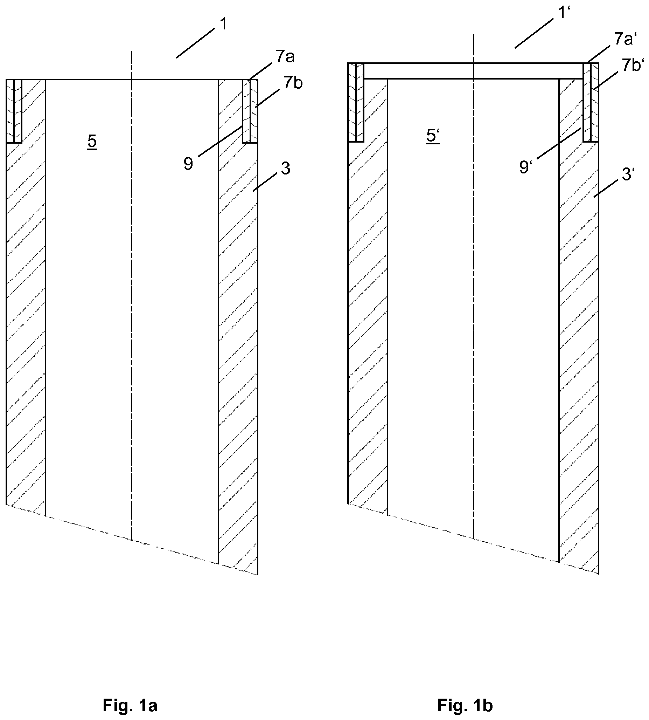

Figure 1a shows a sectional view of an immersion probe 1 for use in a converter steelmaking process according to a first embodiment of the invention. The shown immersion probe 1 comprises a tube 3 having a mounting portion 5 for mounting the tube 3 to a lance (not shown in figure 1a). In the shown embodiment the mounting portion 5 is simply an end portion of the tube 3. A coupling tap on an immersion end of the lance can be pushed into the end portion for attaching the tube 3 to the lance. Sensing elements and/or samplers (not shown in figure 1a) can be arranged on a second end of the tube 3 which is opposite the first end where the mounting portion 5 is located.

Also, shown in figure 1a are two elastic ring seals 7a, 7b that are arranged around the mounting portion 5 of the tube 3. As it can be seen from the figure, the two elastic ring seals 7a, 7b are arranged circumferentially on top each other in a recess 9 which is shown in this embodiment as a groove in the material of the tube 3 and that extends fully around the circumference of the tube 3. In the shown embodiment neither of the two elastic ring seals 7a, 7b projects beyond an end of the tube 3. Also, it can be seen that both elastic ring seals 7a, 7b radially flush with an outer surface of the tube 3. The elastic ring seals 7a, 7b can be held in place by means of their own tension, by means of glue, or by means of at least one staple.

Figure 1b shows a sectional view of an immersion probe 1' for use in a converter steelmaking process according to a second embodiment of the invention.

The tube 3' can be the tube that is shown in figure 1a. The shown embodiment distinguishes from the embodiment shown in figure 1a in that the two elastic ring seals 7a', 7b' extend beyond an end of the tube 3'. For example, the two elastic ring seals 7a', 7b' can extend beyond the end of the tube 3' by 2 to 7 mm, preferably by 3 to 6 mm, more preferably by 4 to 5 mm.

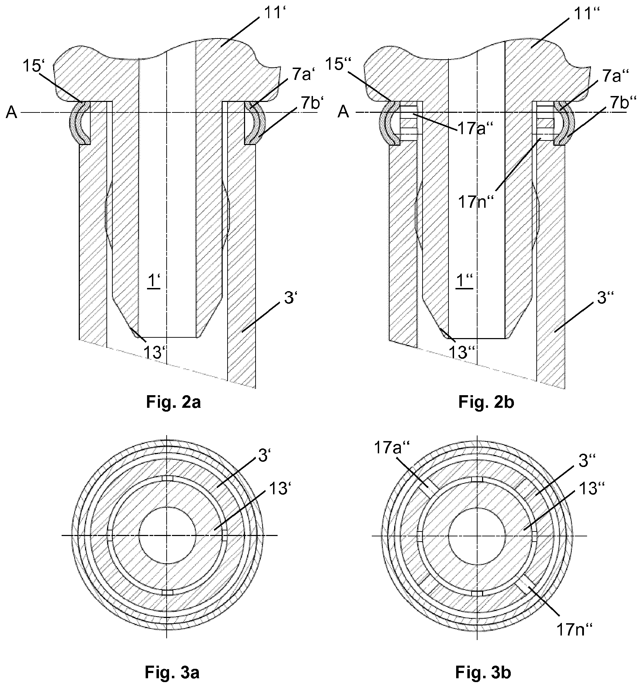

Figure 2a shows a sectional view of a measurement system for use in a converter steelmaking process comprising a lance 11' and an immersion probe 1' according to the first embodiment of the invention mounted onto the lance 11'.

Figure 2a shows the immersion end of the lance 11' with the coupling tap 13'. The part of the lance 11' penetrating in the tube 3' of the immersion probe 1' is commonly known as the coupling tap 13' and also sometimes referred to as lance-holder. The coupling tap 13' has been foreseen with a shoulder 15' that is dimensioned in such a way that the tube 3' can be connected by a push-on force onto the lance 11' and stays in place during the immersion process. Afterwards, the immersion probe 1' can be removed from the lance 11' by applying a pull-of force.

The coupling tap 13' can have a diameter that essentially corresponds to the inner diameter of the tube 3' so that the tube 3' can be pushed onto the coupling tap 13' and held by the resulting force fit connection. In the shown embodiment, the coupling tap 13' has an essentially conical shape with a through hole in the center. The through hole can accommodate wires to connect sensing means on the second end of the tube 3' (not shown in figure 2) to analyzing means (not shown in figure 2) associated with the sensing means. However, in embodiments that are not explicitly shown in here, the coupling tap 13' can also have a different geometrical shape.

The space between the coupling tap 13' and the end portion of the immersion probe 1' is shown as shoulder 15' which is an essentially planar surface which surrounds the coupling tap 13'. When the tube 3' is mounted onto the lance 11', as shown in the figure, the elastic ring seals 7a', 7b' are compressed and rest against the shoulder 15' and, thus, seal the space between the coupling tap 13' and the end portion of the immersion probe 1'.

Figure 2b shows a sectional view of a measurement system for use in a converter steelmaking process comprising a lance 11" and an immersion probe 1" according to a third embodiment of the invention mounted onto the lance 11".

The lance 11" can be the lance that is shown in figure 2a. The immersion probe 1" distinguishes from the immersion probe that is shown in figures 1b and 2a in that the tube 3" has ventilation openings 17a" - 17n" extending through the material of the tube 3" and being arranged in the recess and at least partly covered by the two elastic ring seals 7a", 7b". In the embodiment of figure 3 the ventilation openings 17a" - 17n" are shown as circular holes through the material of the tube 3".

In alternative embodiments the tube can comprise more or fewer ventilation openings than shown in figure 2b. The ventilation openings can also have different shapes, such as having a rectangular shape. Circular holes are considered the best choice from an economical point of view. The number and size of the ventilation openings 17a" - 17n" is chosen based on the desired level of ventilation. This desired level of ventilation depends on the quantity of liquid metal entering the space surrounded by the inner diameter of the tube 3". For example, immersion probes that don't carry a sampling unit only need 1 or 2 ventilation openings of approximately 6 mm, while immersion probes carrying a sampling unit and a liquidus unit will need approximately 6 - 8 ventilation openings of approximately 6 - 8 mm. Most immersion lances are equipped with gas purging and oblige for a minimum level of two ventilation openings.

Figures 3a, 3b show cross-sectional views taken along section lines 'A' as shown in Figures 2a and 2b. Figure 3a shows the tube 3' as shown in figure 2a arranged around the coupling tap 13'. Figure 3b shows ventilation openings 17a" - 17n" arranged in the material of the tube 3".

The features disclosed in the claims, the specification, and the drawings maybe essential for different embodiments of the claimed invention, both separately and in any combination with each other.

### Reference Signs

- 1, 1', 1": Immersion Probe
- 3, 3', 3": Tube
- 5, 5': Mounting Portion
- 7a, 7a', 7a", 7b, 7b', 7b": Elastic Ring Seals
- 9,9': Recess
- 11', 11": Lance
- 13', 13": Coupling Tap
- 15', 15": Shoulder
- 17a" - 17n": Ventilation Openings

- A: Section Line

## Claims

1. An immersion probe (1, 1', 1") for use in a converter steelmaking process, comprising: a tube (3, 3', 3") having a mounting portion (5, 5') for mounting the tube (3, 3', 3") to a lance (11', 11"),
wherein the mounting portion (5, 5') is adapted to couple to a coupling tap (13', 13") on an immersion end of the lance (11', 11"); and two elastic ring seals (7a, 7a', 7a", 7b. 7b', 7b"),
wherein the two elastic ring seals (7a, 7a', 7a", 7b. 7b', 7b") are arranged around the mounting portion (5, 5') of the tube (3, 3', 3"),
wherein the two elastic ring seals (7a, 7a', 7a", 7b. 7b', 7b") are arranged circumferentially on top of each other for sealing a space between the coupling tap (13', 13") and the end portion of the immersion probe (1, 1', 1"), and
wherein the outer circumference of an inner elastic ring seal which is in direct contact with the tube is at least partly in contact with the inner circumference of an outer elastic ring seal so that the outer elastic ring is configured to at least partly cover the inner elastic ring.

2. The immersion probe (1, 1', 1") according to claim 1, wherein the mounting portion (5, 5') comprises a cylindrical opening for accommodating the coupling tap (13', 13"), preferably the mounting portion (5, 5') is an end portion of the tube (3, 3', 3").

3. The immersion probe (1, 1', 1") according to claims 1 or 2, wherein the two elastic ring seals (7a, 7a', 7a", 7b. 7b', 7b") are arranged at least partly in a recess (9, 9') around the mounting portion (5, 5') of the tube (3, 3', 3").

4. The immersion probe (1, 1', 1") according to claim 3, wherein the tube (3, 3', 3") comprises at least one ventilation opening (17a",17n") extending through the material of the tube (3, 3', 3") and arranged in the recess (9, 9') and at least partly covered by at least one of the two elastic ring seals (7a, 7a', 7a", 7b. 7b', 7b").

5. The immersion probe (1, 1', 1") according to claim 4, wherein the tube (3, 3', 3") comprises two to six ventilation openings (17a", 17n"), preferably the ventilation openings (17a",17n") are having a diameter of 5 to 8 mm and are arranged in the recess (9, 9').

6. The immersion probe (1, 1', 1") according to claims 3 to 5, wherein the two elastic ring seals (7a, 7a', 7a", 7b. 7b', 7b") are adapted to radially flush with an outer surface of the tube (3, 3', 3").

7. The immersion probe (1, 1', 1") according to any of the preceding claims, wherein at least one of the elastic ring seals is adapted to extend beyond an end of the tube (3, 3', 3"), preferably the two elastic ring seals (7a, 7a', 7a", 7b. 7b', 7b") are adapted to extend beyond the end of the tube (3, 3', 3").

8. The immersion probe (1, 1', 1") according to claim 7, wherein at least one elastic ring seal, preferably the two elastic ring seals (7a, 7a', 7a", 7b. 7b', 7b"), are adapted to extend beyond an end of the tube (3, 3', 3") by 2 to 7 mm, preferably by 3 to 6 mm, more preferably by 4 to 5 mm.

9. The immersion probe (1, 1', 1") according to any of the preceding claims, wherein the tube (3, 3', 3") comprises at least a cardboard material.

10. The immersion probe (1, 1', 1") according to any of the preceding claims, wherein the tube (3, 3', 3") having an outer diameter of 80 mm.

11. The immersion probe (1, 1', 1") according to any of the preceding claims, wherein the two elastic ring seals (7a, 7a', 7a", 7b. 7b', 7b") comprise a rubber material.

12. The immersion probe (1, 1', 1") according to claim 11, wherein the two elastic ring seals (7a, 7a', 7a", 7b. 7b', 7b") comprise a material comprising ethylene propylene diene monomer, EPDM, rubber.

13. The immersion probe (1, 1', 1") according to any of the preceding claims, wherein the two elastic ring seals (7a, 7a', 7a", 7b. 7b', 7b") have a length of 25 mm, and/or a thickness of 2.5 mm.

14. A measuring system for use in a converter steelmaking process comprising: a lance (11', 11") comprising an immersion end with a coupling tap (13', 13"); and an immersion probe (1, 1', 1") according to claim 1.

## Patentansprüche

1. Tauchsonde (1, 1', 1") zur Verwendung in einem
Konverterstahlerzeugungsverfahren, umfassend: ein Rohr (3, 3', 3") aufweisend einen Befestigungsabschnitt (5, 5') zum Befestigen des Rohres (3, 3', 3") an einer Lanze (11', 11"),
wobei der Befestigungsabschnitt (5, 5') so ausgelegt ist, dass er mit einem Kopplungsgewindebohrer (13', 13") an einem Eintauchende der Lanze (11', 11") gekoppelt werden kann; und zwei elastische Ringdichtungen (7a, 7a', 7a", 7b, 7b', 7b"), wobei die zwei elastischen Ringdichtungen (7a, 7a', 7a", 7b, 7b', 7b") um den Befestigungsabschnitt (5, 5') des Rohres (3, 3', 3") herum angeordnet sind,
wobei die beiden elastischen Ringdichtungen (7a, 7a', 7a", 7b, 7b', 7b") in Umfangsrichtung übereinander angeordnet sind, um einen Raum zwischen dem Kopplungsgewindebohrer (13', 13") und dem Endabschnitt der Tauchsonde (1, 1', 1") abzudichten, und
wobei der Außenumfang einer inneren elastischen Ringdichtung, die in direktem Kontakt mit dem Rohr steht, mindestens teilweise in Kontakt mit dem Innenumfang einer äußeren elastischen Ringdichtung steht, so dass der äußere elastische Ring so konfiguriert ist, dass er den inneren elastischen Ring mindestens teilweise bedeckt.

2. Tauchsonde (1, 1', 1") nach Anspruch 1, wobei der Befestigungsabschnitt (5, 5') eine zylindrische Öffnung zur Aufnahme des Kopplungsgewindebohrers (13', 13") aufweist, wobei bevorzugt der Befestigungsabschnitt (5, 5') ein Endabschnitt des Rohres (3, 3', 3") ist.

3. Tauchsonde (1, 1', 1") nach Anspruch 1 oder 2, wobei die beiden elastischen Ringdichtungen (7a, 7a', 7a", 7b, 7b', 7b") mindestens teilweise in einer Aussparung (9, 9') um den Befestigungsabschnitt (5, 5') des Rohres (3, 3', 3") herum angeordnet sind.

4. Tauchsonde (1, 1', 1") nach Anspruch 3, wobei das Rohr (3, 3', 3") mindestens eine Belüftungsöffnung (17a", 17n") aufweist, die sich durch das Material des Rohres (3, 3', 3") erstreckt und in der Aussparung (9, 9') angeordnet ist und mindestens teilweise von mindestens einer der beiden elastischen Ringdichtungen (7a, 7a', 7a", 7b, 7b', 7b") bedeckt ist.

5. Tauchsonde (1, 1', 1") nach Anspruch 4, wobei das Rohr (3, 3', 3") zwei bis sechs Belüftungsöffnungen (17a", 17n") aufweist, wobei die Belüftungsöffnungen (17a", 17n") bevorzugt einen Durchmesser von 5 bis 8 mm haben und in der Aussparung (9, 9') angeordnet sind.

6. Tauchsonde (1, 1', 1") nach den Ansprüchen 3 bis 5, wobei die beiden elastischen Ringdichtungen (7a, 7a', 7a", 7b, 7b', 7b") so ausgelegt sind, dass sie radial bündig mit einer Außenfläche des Rohres (3, 3', 3") abschließen.

7. Tauchsonde (1, 1', 1") nach einem der vorhergehenden Ansprüche, wobei mindestens eine der elastischen Ringdichtungen so ausgelegt ist, dass sie sich über ein Ende des Rohres (3, 3', 3") hinaus erstreckt, wobei bevorzugt die beiden elastischen Ringdichtungen (7a, 7a', 7a", 7b, 7b', 7b") so ausgelegt sind, dass sie sich über das Ende des Rohres (3, 3', 3") hinaus erstrecken.

8. Tauchsonde (1, 1', 1") nach Anspruch 7, wobei mindestens eine elastische Ringdichtung, bevorzugt die beiden elastischen Ringdichtungen (7a, 7a', 7a", 7b, 7b', 7b"), so ausgelegt sind, dass sie sich über ein Ende des Rohres (3, 3', 3") um 2 bis 7 mm, bevorzugt um 3 bis 6 mm, stärker bevorzugt um 4 bis 5 mm, hinaus erstrecken.

9. Tauchsonde (1, 1', 1") nach einem der vorhergehenden Ansprüche, wobei das Rohr (3, 3', 3") mindestens aus einem Kartonmaterial besteht.

10. Tauchsonde (1, 1', 1") nach einem der vorhergehenden Ansprüche, wobei das Rohr (3, 3', 3") einen Außendurchmesser von 80 mm aufweist.

11. Tauchsonde (1, 1', 1") nach einem der vorhergehenden Ansprüche, wobei die beiden elastischen Ringdichtungen (7a, 7a', 7a", 7b, 7b', 7b") aus einem Gummimaterial bestehen.

12. Tauchsonde (1, 1', 1") nach Anspruch 11, wobei die beiden elastischen Ringdichtungen (7a, 7a', 7a", 7b, 7b', 7b") ein Material umfassen, das Ethylen-Propylen-Dien-Monomer, EPDM, Kautschuk umfasst.

13. Tauchsonde (1, 1', 1") nach einem der vorhergehenden Ansprüche, wobei die beiden elastischen Ringdichtungen (7a, 7a', 7a", 7b, 7b', 7b") eine Länge von 25 mm und/oder eine Dicke von 2,5 mm aufweisen.

14. Messsystem zur Verwendung in einem Konverterstahlerzeugungsverfahren, umfassend: eine Lanze (11', 11"), die ein Eintauchende mit einem Kopplungsgewindebohrer (13', 13") umfasst; und eine Tauchsonde (1, 1', 1") nach Anspruch 1.

## Revendications

1. Une sonde d'immersion (1, 1', 1") destinée à être utilisée dans un procédé d'aciérie pour convertisseur, comprenant : un tube (3, 3', 3") ayant une partie de montage (5, 5') pour le montage du tube (3, 3', 3") sur une lance (11', 11"),
la partie de montage (5, 5') étant adaptée pour être raccordée à un robinet de raccordement (13', 13") sur une extrémité d'immersion de la lance (11', 11") et deux joints d'étanchéité élastiques (7a, 7a', 7a", 7b. 7b', 7b"),
les deux joints d'étanchéité élastiques (7a, 7a', 7a", 7b. 7b', 7b") étant disposés autour de la partie de montage (5, 5') du tube (3, 3', 3"),
les deux joints d'étanchéité élastiques (7a, 7a', 7a", 7b. 7b', 7b") étant disposés sur la circonférence en haut l'un de l'autre pour étanchéifier un espace entre le robinet de raccordement (13', 13") et la partie d'extrémité de la sonde d'immersion (1, 1', 1") et
la circonférence extérieure d'un joint d'étanchéité élastique intérieur qui est en contact direct avec le tube étant au moins partiellement en contact avec la circonférence intérieure d'un joint d'étanchéité élastique extérieur de telle manière à ce que le joint élastique extérieur soit configuré pour couvrir au moins partiellement le joint élastique intérieur.

2. La sonde d'immersion (1, 1', 1") conformément à la revendication 1, dans laquelle la partie de montage (5, 5') comprend une ouverture cylindrique pour accueillir le robinet de raccordement (13', 13"), la partie de montage (5, 5') étant préférablement une partie d'extrémité du tube (3, 3', 3").

3. La sonde d'immersion (1, 1', 1") conformément aux revendications 1 ou 2, dans laquelle les deux joints d'étanchéité élastiques (7a, 7a', 7a", 7b. 7b', 7b") sont disposés au moins partiellement dans un renfoncement (9, 9') autour de la partie de montage (5, 5') du tube (3, 3', 3").

4. La sonde d'immersion (1, 1', 1") conformément à la revendication 3, dans laquelle le tube (3, 3', 3") comprend au moins une ouverture de ventilation (17a", 17n") s'étendant à travers le matériau du tube (3, 3', 3") et disposée dans le renfoncement (9, 9') et étant au moins partiellement couverte par au moins l'un des deux joints d'étanchéité élastiques (7a, 7a', 7a", 7b. 7b', 7b").

5. La sonde d'immersion (1, 1', 1") conformément à la revendication 4, dans laquelle le tube (3, 3', 3") comprend deux à six ouvertures de ventilation (17a", 17n"), les ouvertures de ventilation (17a", 17n") ayant préférablement un diamètre de 5 à 8 mm et étant disposées dans le renfoncement (9, 9').

6. La sonde d'immersion (1, 1', 1") conformément aux revendications 3 à 5, dans laquelle les deux joints d'étanchéité élastiques (7a, 7a', 7a", 7b. 7b', 7b") sont adaptés pour s'aligner dans le sens radial avec une surface extérieure du tube (3, 3', 3").

7. La sonde d'immersion (1, 1', 1") conformément à l'une des revendications précédentes, dans laquelle au moins l'un des joints d'étanchéité élastiques est adapté pour s'étendre au-delà d'une extrémité du tube (3, 3', 3"), les deux joints d'étanchéité élastiques (7a, 7a', 7a", 7b. 7b', 7b") étant de préférence adaptés pour s'étendre au-delà de l'extrémité du tube (3, 3', 3").

8. La sonde d'immersion (1, 1', 1") conformément à la revendication 7, dans laquelle au moins un joint d'étanchéité élastique, préférablement les deux joints d'étanchéité élastiques (7a, 7a', 7a", 7b. 7b', 7b") sont adaptés pour s'étendre au-delà d'une extrémité du tube (3, 3', 3") de 2 à 7 mm, préférablement de 3 à 6 mm, plus préférablement de 4 à 5 mm.

9. La sonde d'immersion (1, 1', 1") conformément à l'une des revendications précédentes, dans laquelle le tube (3, 3', 3") comprend au moins une matière en carton.

10. La sonde d'immersion (1, 1', 1") conformément à l'une des revendications précédentes, dans laquelle le tube (3, 3', 3") a un diamètre extérieur de 80 mm.

11. La sonde d'immersion (1, 1', 1") conformément à l'une des revendications précédentes, dans laquelle les deux joints d'étanchéité élastiques (7a, 7a', 7a", 7b. 7b', 7b") comprennent un matériau en caoutchouc.

12. La sonde d'immersion (1, 1', 1") conformément à la revendication 11, dans laquelle les deux joints d'étanchéité élastiques (7a, 7a', 7a", 7b. 7b', 7b") comprennent un matériau contenant du caoutchouc EPDM (éthylène-propylène-diène monomère).

13. La sonde d'immersion (1, 1', 1") conformément à l'une des revendications précédentes, dans laquelle les deux joints d'étanchéité élastiques (7a, 7a', 7a", 7b. 7b', 7b") ont une longueur de 25 mm et/ou une épaisseur de 2,5 mm.

14. Un système de mesure destiné à être utilisé dans un procédé d'aciérie pour convertisseur comprenant : une lance (11', 11") comprenant une extrémité d'immersion avec un robinet d'accouplement (13', 13") et une sonde d'immersion (1, 1', 1") conformément à la revendication 1.
